# FASCICULE DE BREVET EUROPEEN

(11) **EP 1 909 712 B1**
(45) Date de publication et mention de la délivrance du brevet: **25.08.2010**
(21) Numéro de dépôt: 05775760.1
(22) Date de dépôt: 01.06.2005
(51) Int. Cl.: A61F 5/00

(54) **BALLON INTRA-GASTRIQUE POURVU D'UNE VALVE CONTENANT UN GEL ET KIT**
INTRAGASTRISCHER BALLON MIT EINEM GELHALTIGEN VENTIL UND KIT
INTRA-GASTRIC BALLOON PROVIDED WITH A GEL-CONTAINING VALVE AND KIT

(43) Date de publication de la demande: 16.04.2008
(73) Titulaire: Compagnie Européenne d' Etude et de Recherche de Dispositifs pour L'Implantation par Laparoscopie, 38200 Vienne (FR)
(72) Inventeur: VALENCON, Nicolas, François, Michel, F-38780 Pont Eveque (US); PAGANON, Pascal, F-69360 Serezin du Rhône (FR)
(74) Mandataire: Croonenbroek, Thomas Jakob
(86) Numéro de dépôt international: PCT/FR2005/001345
(87) Numéro de publication internationale: WO 2006/128979

(56) Documents cités:
- WO-A-03/055420
- FR-A- 2 861 288
- FR-A- 2 862 525

## Description

### DOMAINE TECHNIQUE

La présente invention se rapporte au domaine technique général des dispositifs implantables dans le corps humain destinés à être utilisés dans le cadre d'un traitement de l'obésité et notamment de l'obésité morbide, et tout particulièrement à des implants aptes à réduire artificiellement le volume de l'estomac en vue notamment de produire une sensation de satiété chez le patient.

La présente invention concerne plus particulièrement un ballon intra-gastrique destiné à être implanté à l'intérieur de l'estomac d'un patient pour réduire le volume de l'estomac dans le cadre d'un traitement de l'obésité, ledit ballon intra-gastrique comprenant :
- au moins une poche apte à être remplie, au moins partiellement, avec un fluide de gonflage,
- un moyen de connexion, monté sur ladite poche et apte à recevoir un organe de connexion à une source de fluide de gonflage.

La présente invention concerne également un kit de mise en forme d'un ballon intra-gastrique destiné à être implanté au sein de l'estomac d'un patient dans le cadre d'un traitement de l'obésité comprenant :
- un ballon intra-gastrique,
- un organe de connexion du ballon intra-gastrique à une source de fluide de gonflage.

La présente invention concerne également une nouvelle utilisation d'un fluide visqueux au sein d'un moyen de connexion d'un ballon intra-gastrique à une source de fluide.

### TECHNIQUE ANTERIEURE

Pour traiter les patients atteints d'obésité, il est connu d'utiliser des ballons intra-gastriques destinés à être implantés dans l'estomac des patients pour diminuer l'espace disponible pour les aliments, en vue notamment de produire une sensation de satiété.

Les ballons intra-gastriques connus comportent généralement au moins une poche souple apte à occuper une configuration repliée (ou à volume réduit) permettant l'implantation du ballon intra-gastrique par voie orale.

La poche est ensuite destinée à être remplie, une fois le ballon intra-gastrique implanté dans l'estomac, à l'aide d'un fluide de gonflage, et par exemple à l'aide d'air ou de liquide physiologique de manière à conférer au ballon intra-gastrique sa forme fonctionnelle, dans laquelle il occupe un volume suffisant au sein de l'estomac pour occuper une partie importante de l'espace disponible pour les aliments.

La poche est généralement réalisée à partir de matériaux présentant de bonnes propriétés d'étanchéité de manière à éviter la fuite progressive du fluide contenu dans la poche vers l'estomac et le dégonflage du ballon intra-gastrique. En particulier, l'étanchéité de la poche doit être suffisante pour que le ballon intra-gastrique conserve sa forme fonctionnelle pendant toute la durée du traitement qui peut varier de quelques semaines à plusieurs mois.

Dans les ballons intra-gastriques connus, la poche comporte un moyen de connexion, tel qu'une valve, destiné à permettre le raccordement du ballon intra-gastrique à une source de fluide de gonflage, par l'intermédiaire d'un organe de connexion comprenant par exemple un cathéter et une aiguille de gonflage. Le moyen de connexion comprend en outre des moyens assurant son étanchéité, une fois l'organe de connexion retiré, afin d'éviter la fuite du fluide contenu dans la poche.

Dans le cas où le fluide de gonflage est formé par un liquide, tel que du liquide physiologique, les moyens utilisés peuvent se présenter sous la forme d'une valve *« en bec de canard »*. Les valves en « *bec de canard* » sont conçues de manière à autoriser le passage du fluide sous pression de l'extérieur vers l'intérieur du ballon intra-gastrique et à interdire le passage du fluide dans le sens inverse. Ces valves permettent en règle générale d'obtenir de bons résultats avec les liquides, mais ne donnent généralement pas satisfaction lorsque le fluide de gonflage est formé par un gaz. En effet, l'étanchéité procurée par ce type de valves n'est alors pas satisfaisante. On préfère alors utiliser des valves du type *« septum »,* formées par une membrane plus ou moins épaisse en silicone, apte à être transpercée par l'aiguille de gonflage et présentant un caractère auto-cicatrisant.

Les ballons intra-gastriques équipés avec les valves du type *« septum »* sont de fabrication simple et donnent généralement satisfaction, mais présentent toutefois plusieurs inconvénients non négligeables.

Tout d'abord, en cas de stockage prolongé, un phénomène de vieillissement du silicone, formant le septum, peut apparaître. En particulier, le septum peut devenir sec, cassant et perdre son élasticité et ses propriétés d'étanchéité sous l'effet du vieillissement.

Par ailleurs, les ballons intra-gastriques sont parfois vendus au sein d'un kit comprenant une aiguille de gonflage pré-installée sur le ballon, l'aiguille de gonflage étant pré-positionnée, dans sa position fonctionnelle, de manière à traverser le septum et à déboucher dans la poche du ballon intra-gastrique.

De tels kits peuvent être stockés au sein de boîtiers stériles ou non stériles pendant une période prolongée avant d'être utilisés. Or, la présence prolongée de l'aiguille de gonflage à travers le septum peut conduire à la formation d'un orifice qui, si la durée de stockage est importante, peut persister après le retrait de l'aiguille, une fois le ballon intra-gastrique gonflé. Cet orifice constitue alors un passage préférentiel par lequel le fluide contenu dans le ballon intra-gastrique est susceptible de s'échapper, entraînant alors le dégonflage du ballon.

Un ballon intra-gastrique selon le préambule de la revendications 1 est connu du document FR-A-2 862 525.

### EXPOSE DE L'INVENTION

Les objets assignés à l'invention visent en conséquence à porter remède aux différents inconvénients énumérés précédemment et à proposer un nouveau ballon intra-gastrique dont l'étanchéité est supérieure à celle des ballons intra-gastriques de l'art antérieur, même après une période de stockage prolongée.

Un autre objet de l'invention vise à proposer un nouveau ballon intra-gastrique susceptible d'être connecté facilement, de manière étanche et en toute sécurité à une source de fluide de gonflage.

Un autre objet de l'invention vise à proposer un nouveau ballon intra-gastrique qui soit particulièrement léger et bien supporté par le patient.

Un autre objet de l'invention vise à proposer un nouveau ballon intra-gastrique, dont l'extraction peut être réalisée facilement et rapidement, par voie orale.

Un autre objet de l'invention vise à proposer un nouveau ballon intra-gastrique susceptible d'être facilement et solidement agrippé par des outils d'extraction endoscopiques.

Les objets assignés à l'invention visent également à proposer un nouveau kit comprenant un ballon intra-gastrique et un organe de connexion du ballon à une source de fluide de gonflage présentant, même après une longue période de stockage, de bonnes propriétés d'étanchéité.

Les objets assignés à l'invention visent également à proposer une nouvelle utilisation d'un fluide visqueux permettant d'améliorer l'étanchéité d'un ballon intra-gastrique.

Les objets assignés à l'invention sont atteints à l'aide d'un ballon intra-gastrique destiné à être implanté à l'intérieur de l'estomac d'un patient pour réduire le volume de l'estomac dans le cadre d'un traitement de l'obésité, ledit ballon intra-gastrique comprenant :
- au moins une poche apte à être remplie, au moins partiellement, avec un fluide de gonflage,
- un moyen de connexion, monté sur ladite poche et apte à recevoir
   un organe de connexion à une source de fluide de gonflage, caractérisé en ce que le moyen de connexion comporte une chambre conçue pour être transpercée par ledit organe de connexion en vue d'injecter le fluide de gonflage dans ladite poche, ladite chambre renfermant un fluide d'étanchéité suffisamment visqueux et/ou consistant pour assurer l'étanchéité du moyen de connexion une fois l'organe de connexion retiré.
   Les objets assignés à l'invention sont également atteints à l'aide d'un kit de mise en forme d'un ballon intra-gastrique au sein de l'estomac d'un patient dans le cadre d'un traitement de l'obésité comprenant :
- un ballon intra-gastrique conforme à l'invention, et notamment tel que décrit précédemment,
- un organe de connexion du ballon intra-gastrique à la source de fluide de gonflage, monté, dans sa configuration de stockage, de manière à traverser de part en part ladite chambre pour déboucher dans la poche.

Les objets assignés à l'invention sont également atteints à l'aide d'une utilisation d'un fluide visqueux et/ou consistant en tant que joint d'étanchéité au sein d'un moyen de connexion, monté sur un ballon intra-gastrique et conçu pour permettre le raccordement étanche du ballon intra-gastrique à une source de fluide de gonflage.

### DESCRIPTIF SOMMAIRE DES DESSINS

D'autres objets et avantages de l'invention apparaîtront plus en détails à la lecture de la description qui suit, ainsi qu'à l'aide des dessins annexés ci-après, donnés à titre purement illustratif et non limitatif, dans lesquels :
- La figure 1 illustre, selon une vue en coupe, un ballon intra-gastrique conforme à l'invention, comportant une seule poche.
- La figure 2 illustre, selon une vue en coupe, une variante de réalisation d'un ballon intra-gastrique conforme à l'invention comportant deux poches concentriques.
- La figure 3 illustre, selon une vue en perspective, un moyen de connexion conforme à l'invention destiné à être monté sur un ballon intra-gastrique.
- La figure 4 illustre, selon une vue en coupe suivant la ligne A-A illustrée sur la figure 3, le moyen de connexion illustré sur la figure 3.

### MEILLEURE MANIERE DE REALISER L'INVENTION

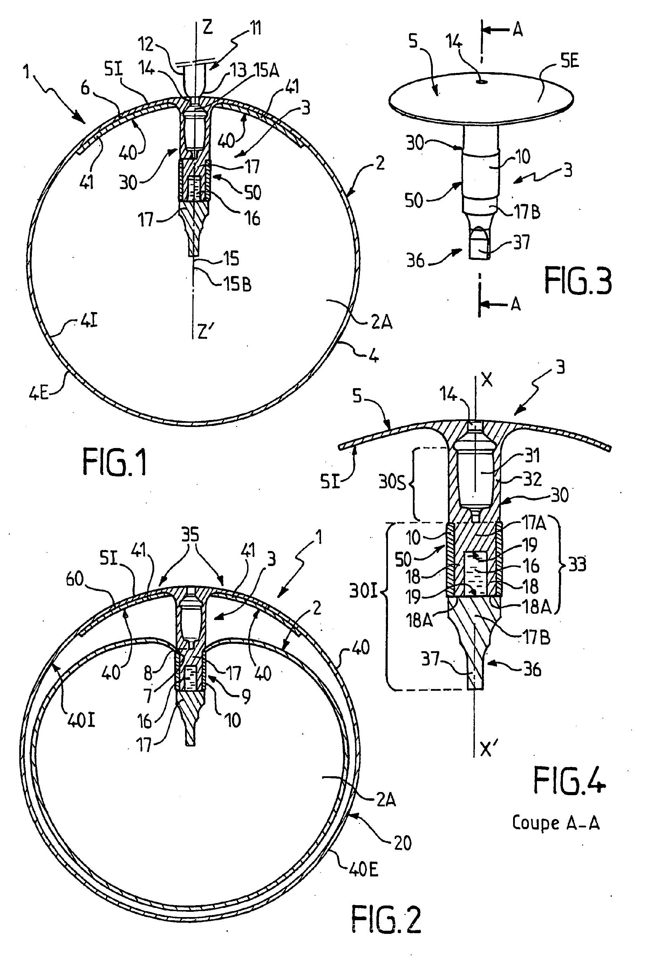

Les figures 1 et 2 illustrent deux variantes de réalisation d'un ballon intra-gastrique 1 conforme à l'invention dans sa configuration fonctionnelle, c'est-à-dire expansée, dans laquelle il occupe une partie importante de l'espace disponible pour les aliments au sein de l'estomac.

Le ballon intra-gastrique 1 conforme à l'invention présente avantageusement un caractère expansible, c'est-à-dire qu'il est réalisé à partir de matériaux souples et de préférence à partir de matériaux élastomères tels que le silicone ou le polyuréthane thermoplastique élastomère, permettant son expansion entre une configuration repliée (non représentée) dans laquelle il occupe un faible volume permettant son implantation par voie orale, et d'autre part une configuration expansée dans laquelle il occupe un volume fonctionnel (figures 1 et 2).

Le ballon intra-gastrique 1 conforme à l'invention comprend au moins une poche 2 apte à être remplie, au moins partiellement, avec un fluide de gonflage et délimitant un volume interne 2A.

Le ballon intra-gastrique 1 conforme à l'invention comprend également un moyen de connexion 3, monté sur ladite poche 2 et destiné à être relié à une source de fluide (non représentée) à l'aide d'un organe de connexion 11 pour assurer l'expansion du ballon intra-gastrique 1 dans l'estomac par remplissage avec le fluide de gonflage.

Selon un premier mode de réalisation de l'invention illustré sur la figure 1, le ballon intra-gastrique 1 comprend une unique poche 2 formant une enveloppe superficielle 4 pour le ballon intra-gastrique 1. De façon préférentielle, la poche 2 est réalisée à partir d'un matériau souple biocompatible, et de préférence à partir de silicone de grade médical.

Selon ce premier mode de réalisation, le moyen de connexion 3 est monté sur la poche 2 et de préférence solidarisé avec cette dernière, par exemple par collage ou soudure. Le moyen de connexion 3 comporte à cet effet une collerette 5 destinée à permettre la fixation étanche du moyen de connexion 3 sur la poche 2, par exemple par collage ou soudure de la collerette 5 sur le pourtour d'une lumière 6 ménagée à travers la poche 2.

Selon un deuxième mode de réalisation illustré sur la figure 2, le ballon intra-gastrique 1 conforme à l'invention comprend au moins une première et une deuxième poches 2, 20 préférentiellement souples, la première poche 2 étant disposée à l'intérieur de la deuxième poche 20 de manière à former une première poche 2 interne et une deuxième poche 20 externe. Selon cette variante, le moyen de connexion 3 est fixé de façon étanche sur la première poche 2 et plus précisément monté au sein d'un passage 7 délimité par un col 8 s'étendant vers l'intérieur de la première poche 2. Plus précisément, le moyen de connexion 3 est fixé sur la première poche 2 à l'aide d'un élément de fixation 9 préférentiellement formé par une bague 10. L'élément de fixation 9 est ainsi avantageusement conformé et disposé pour exercer une pression suffisante sur le col 8 pour le pincer entre le moyen de connexion 3 et l'élément de fixation 7.

Selon ce deuxième mode de réalisation de l'invention, la première poche 2 forme un moyen de mise en forme de la deuxième poche 20, cette dernière formant alors l'enveloppe superficielle 40 du ballon intra-gastrique 1. Le fluide de gonflage et notamment l'air est avantageusement introduit dans la première poche 2 engendrant ainsi son gonflage. L'expansion de la première poche 2 engendre à son tour, à la manière d'une *« chambre à air »* l'expansion et la mise en forme de la deuxième poche 20 formant l'enveloppe superficielle 40 du ballon intra-gastrique 1.

Avantageusement, la première et la deuxième poches 2, 20 sont réalisées à partir de matériaux différents et non nécessairement compatibles.

L'expression « *non compatibles »* fait référence à des matériaux dont l'assemblage classique par collage ou par soudure s'avèrerait particulièrement difficile, voire impossible compte-tenu des contraintes de production industrielle et des exigences médicales en matière de qualité et de sécurité.

De façon préférentielle, les première et deuxième poches 2, 20 sont réalisées à partir de matériaux élastomères, la première poche 2 étant de préférence réalisée à partir d'un matériau à effet barrière aux gaz, tel que le polyuréthane thermoplastique élastomère et la deuxième poche 20 étant de préférence réalisée à partir d'un matériau biocompatible et présentant une bonne résistance mécanique, tel que le silicone. L'utilisation d'un matériau à effet barrière pour former la première poche 2 permet avantageusement de diminuer l'épaisseur de la première poche 2 tout en conservant, voire en améliorant l'étanchéité du ballon.

Selon ce deuxième mode de réalisation de l'invention, le pourtour de la collerette 5 est préférentiellement collé ou soudé avec la lumière 60 ménagée à travers la deuxième poche 20, le moyen de connexion 3 assurant ainsi l'obturation étanche de la deuxième poche 20, c'est-à-dire de l'enveloppe superficielle 40 du ballon intra-gastrique 1. La configuration à deux poches illustrée sur la figure 2 est préférée à la configuration à une poche illustrée sur la figure 1 car elle permet d'obtenir un ballon intra-gastrique 1 présentant une résistance mécanique et une étanchéité supérieures à celles des ballons simple poche, tout en conservant un encombrement faible à l'état plié, grâce notamment à la faible épaisseur de la poche interne.

L'organe de connexion 11 comprend avantageusement un cathéter 12 destiné à assurer la liaison fluidique entre le ballon intra-gastrique 1 et la source de fluide de gonflage et conçu pour être raccordé au ballon intra-gastrique 1 à l'aide d'un embout 13 introduit au sein d'un logement 14 ménagé dans le moyen de connexion 3. L'organe de connexion 11 comprend également une aiguille de gonflage 15, préférentiellement formée par une aiguille creuse, dont l'une des extrémités 15A est solidarisée avec l'embout 13 et l'autre extrémité 15B, préférentiellement atraumatique, est située à l'intérieur du volume interne 2A. Tel que cela est illustré sur la figure 1, l'aiguille de gonflage est introduite à travers le moyen de connexion 3 suivant un axe de perforation Z-Z'.

Selon l'invention, le moyen de connexion 3 comporte une chambre 16 conçue et avantageusement disposée pour être transpercée par l'organe de connexion 11, et plus précisément par l'aiguille de gonflage 15 en vue d'injecter le fluide de gonflage dans la poche 2, ladite chambre 16 renfermant un fluide d'étanchéité suffisamment visqueux et/ou consistant pour assurer l'étanchéité du moyen de connexion 3 une fois l'organe de connexion 11 retiré. Ainsi, après le retrait de l'organe de connexion 11, et notamment de l'aiguille de gonflage 15, le fluide d'étanchéité contenu dans la chambre 16 se déplace et vient occuper l'espace laissé libre par l'aiguille de gonflage 15. La chambre 16, avantageusement entièrement remplie avec le fluide d'étanchéité constitue alors un organe auto-obturant, apte à se refermer automatiquement de façon étanche après le retrait de l'aiguille de gonflage 15.

L'expression « *fluide d'étanchéité »* désigne ici tout type de substance fluide ou semi-solide suffisamment visqueuse et/ou consistante pour assurer une fonction d'étanchéité. Il peut s'agir d'un liquide, caractérisé par sa viscosité ou encore d'un semi-solide, caractérisé par sa consistance et sa résistance à l'enfoncement, mesurée à l'aide d'un pénétromètre. Ainsi, pour certains fluides (ou semi-solides) présentant une consistance et une viscosité élevées, tels que des crèmes ou des gels, pour lesquels les mesures avec un viscosimètre standard sont difficiles et peu fiables, on a souvent recours à des mesures de pénétration, qui consistent à mesurer, à 25°C, l'enfoncement par un mobile de poids et de dimensions déterminées, pendant un temps fixe.

Le moyen de connexion 3 comprend avantageusement un corps principal 30, de préférence sensiblement allongé et faisant saillie vers l'intérieur du ballon intra-gastrique 1, et notamment dans le volume interne 2A. La chambre 16 est alors préférentiellement ménagée au sein du corps principal 30.

Avantageusement, le moyen de connexion 3, et notamment le corps principal 30 comporte au moins une cloison auto-cicatrisante 17 disposée en alignement axial avec la chambre 16 de manière à être transpercée par l'organe de connexion 11, ladite cloison auto-cicatrisante 17 étant suffisamment souple et élastique pour s'auto-obturer une fois l'organe de connexion 11 retiré (figures 1 et 2).

L'expression « *cloison auto-cicatrisante »* fait référence à une paroi ou une membrane en matériau suffisamment souple pour pouvoir être transpercée par l'organe de connexion 11, et notamment par l'aiguille de gonflage 15, et présentant des propriétés d'élasticité et de mémoire de forme suffisantes pour combler l'orifice laissé libre après le retrait de l'aiguille de gonflage 15, formant ainsi une cloison étanche.

De façon particulièrement avantageuse, et tel que cela est illustré sur les figures 1 et 2, le moyen de connexion 3 comporte au moins deux cloisons auto-cicatrisantes 17 disposées de part et d'autre de la chambre 16, en alignement axial avec cette dernière.

Le corps principal 30 s'étend de préférence longitudinalement, vers l'intérieur du volume interne 2A, suivant un axe longitudinal X-X', sensiblement confondu avec l'axe de perforation Z-Z'. Lorsque le ballon intra-gastrique 1 est positionné dans la configuration verticale illustrée sur la figure 1, l'axe longitudinal X-X' coïncide sensiblement avec la direction verticale, le moyen de connexion 3 étant alors situé dans la partie supérieure du ballon intra-gastrique 1.

Avantageusement, et tel quel cela est illustré sur la figure 4, le moyen de connexion 3 comprend une cloison auto-cicatrisante supérieure 17A et une cloison auto-cicatrisante inférieure 17B. Les termes *« inférieur »* et *« supérieur »* font référence au positionnement relatif des cloisons auto-cicatrisantes 17 lorsque le ballon intra-gastrique 1 est dans la configuration verticale illustrée sur la figure 1. Les cloisons auto-cicatrisantes 17 sont préférentiellement réalisées à partir d'un matériau élastomère, tel que du silicone solide et souple de grade médical. Les cloisons auto-cicatrisantes supérieure 17A et inférieure 17B peuvent avantageusement être réalisées à partir de matériaux identiques ou différents, et par exemple à partir de silicones de duretés différentes. De façon préférentielle, l'intégralité du moyen de connexion 3 est en silicone.

La chambre 16 est avantageusement délimitée axialement, c'est-à-dire suivant l'axe longitudinal X-X' d'extension du corps principal 30, par les cloisons auto-cicatrisantes supérieure 17A et inférieure 17B respectivement. La chambre 16 est également délimitée, latéralement, c'est-à-dire suivant un plan sensiblement perpendiculaire à l'axe longitudinal X-X', par une ou plusieurs parois latérales 18.

De façon préférentielle, les parois latérales 18 présentent un caractère étanche et sont de préférence formées par une ou plusieurs membranes auto-cicatrisantes. La chambre 16 est alors isolée de manière étanche vis-à-vis du volume interne 2A et vis-à-vis de l'extérieur du ballon, par des parois étanches destinées à empêcher la communication fluidique entre la chambre 16 et le volume interne 2A d'une part et l'extérieur du ballon intra-gastrique 1 d'autre part.

Avantageusement, la chambre 16 comporte des parois de piquage 19, préférentiellement formées par les cloisons auto-cicatrisantes 17, et notamment par les cloisons auto-cicatrisantes supérieure 17A et inférieure 17B respectivement. L'expression *« parois de piquage »* désigne ici les parois de la chambre 16 au niveau desquelles l'aiguille de gonflage 15 vient transpercer la chambre 16.

Il est bien évidemment envisageable de réaliser une chambre 16 dont les parois, et en particulier les parois de piquage 19 ne sont pas formées par des membranes auto-cicatrisantes. Dans ce cas, le passage ménagé à travers les parois de piquage 19 de la chambre 16 une fois l'organe de connexion 11 retiré devra présenter un diamètre suffisamment faible pour interdire la fuite du fluide d'étanchéité contenu dans la chambré 16. On choisira donc de préférence une aiguille de gonflage 15 particulièrement fine et un fluide d'étanchéité suffisamment visqueux pour rester confiné dans la chambre 16.

Avantageusement, le fluide d'étanchéité présente une viscosité et/ou une consistance suffisante pour piéger les particules de fluide de gonflage, par exemple des bulles de gaz ou des gouttes de liquide physiologique, susceptibles de se former dans la chambre 16 une fois l'organe de connexion 11 retiré. Ainsi, en se retirant, l'organe de connexion 11 et notamment l'aiguille de gonflage 15 libère un passage à travers les parois de piquage 19. Le passage ainsi ménagé peut constituer, s'il n'est pas automatiquement obturé, un orifice de fuite pour le fluide de gonflage contenu dans le volume interne 2A. Ce phénomène peut notamment être observé lorsque les parois de piquage 19 de la chambre 16 ne sont pas formées par des cloisons auto-cicatrisantes 17.

Néanmoins, ce phénomène peut également s'observer dans le cas où la chambre 16 est délimitée par des cloisons auto-cicatrisantes 17, en particulier si l'aiguille de gonflage 15 demeure longtemps à travers les cloisons auto-cicatrisantes 17. Dans ce dernier cas, on peut en effet observer une dégradation du caractère auto-obturant des cloisons auto-cicatrisantes 17.

Le fluide d'étanchéité contenu dans la chambre 16 a alors un rôle essentiel puisqu'il permet d'emprisonner les éventuelles particules de fluide de gonflage susceptibles de s'échapper de la chambre 16 à travers le passage susmentionné.

De façon préférentielle, la viscosité et la consistance du fluide d'étanchéité sont telles que la pénétration à 25°C dudit fluide d'étanchéité par un mobile de 1 pouce et de 12 g, avec un temps de chute de 15s est inférieure à 20mm et de préférence comprise entre 10 et 18 mm.

La pénétration est mesurée par un pénétromètre de type Lab Line 10005 ou équivalent, à 25°C.

De façon encore plus préférentielle, le fluide d'étanchéité est formé par un gel, et par exemple par un gel de silicone du type NuSil de référence MED 12-6300, bi-composant et réticulé après sa mise en place au sein de la chambre 16. La réticulation du gel est préférentiellement effectuée à une température de 140 °C, pendant une durée d'environ 5 heures. Le remplissage de la chambre 16 s'effectue avantageusement en plusieurs étapes, afin de combler progressivement le retrait, chaque étape de remplissage étant suivie d'une étape de réticulation.

Avantageusement, le corps principal 30 comprend une portion supérieure 30S, évidée de manière à former une cavité 31, par exemple cylindrique, et une portion inférieure 30I au sein de laquelle est ménagée la chambre 16.

Il est intéressant de noter que la présence de la cavité 31 au sein du moyen de connexion 3 est indépendante de la présence ou non de la chambre 16. La cavité 31 vise en effet simplement à faciliter la préhension du ballon intra-gastrique 1 lors de son explantation, et pourrait donc être associée avec tout type d'organe auto-obturant, et par exemple avec une simple cloison auto-cicatrisante, telle que la cloison auto-cicatrisante supérieure 17A.

Afin de favoriser la préhension du ballon intra-gastrique 1, la portion supérieure 30S est ainsi évidée sur une distance suffisante pour que la paroi (ou les parois) du corps principal 30 délimitant la cavité 31 puisse se rabattre sur elle-même lors du pincement de la portion supérieure 30S du corps principal 30 avec un outil d'extraction (non représenté) du ballon intra-gastrique 1, tel qu'une pince endoscopique.

De façon préférentielle, la cavité 31 s'étend longitudinalement, c'est-à-dire suivant l'axe longitudinal X-X', sur une longueur supérieure à sa largeur moyenne (ou à son diamètre sans le cas d'une cavité cylindrique) mesurée suivant une direction sensiblement perpendiculaire à l'axe longitudinal X-X'.

A titre d'exemple illustratif et non limitatif, la cavité 31 pourra présenter une longueur supérieure à 1,5 fois sa largeur moyenne.

La longueur de la cavité 31 résulte avantageusement d'un compromis entre :
- d'une part la nécessité de limiter la longueur totale du moyen de connexion 3 et du corps principal 30 pour ne pas gêner l'implantation du ballon intra-gastrique 1 par voie orale,
- et d'autre part la nécessité de pouvoir pincer le moyen de connexion 3 à proximité de l'enveloppe superficielle 4, 40 du ballon intra-gastrique 1 pour permettre l'extraction de ce dernier.

Ainsi, l'implantation du ballon intra-gastrique 1 s'effectue alors que l'aiguille de gonflage 15 est montée sur le moyen de connexion 3 de manière à traverser longitudinalement le corps principal 30. Le corps principal 30 et l'aiguille de gonflage 15 forment alors un ensemble rigide présentant une flexibilité longitudinal très faible. Or, le chirurgien, pour introduire le ballon intra-gastrique 1 dans les voies orales du patient doit pouvoir bénéficier d'une certaine souplesse d'où la nécessité de réduire au minimum la longueur du corps principal 30.

De façon particulièrement avantageuse, la collerette 5 s'étend radialement à la surface du ballon intra-gastrique 1 de manière à former une zone de préhension 35 du ballon intra-gastrique 1 par l'outil d'extraction (non représenté).

Il est envisageable de réaliser la collerette 5 dans un matériau présentant une dureté supérieure à celle du matériau formant l'enveloppe superficielle 4, 40, par exemple à partir d'un silicone dur de manière à offrir, au moins localement, un résistance mécanique supérieure et à éviter son transpercement par l'outil d'extraction. Il est néanmoins préférable, pour permettre le pliage du ballon intra-gastrique 1, de réaliser la collerette 5 à partir d'un matériau souple de dureté équivalente ou à peine supérieure à celle de l'enveloppe superficielle 4, 40.

De façon préférentielle, la zone de préhension 35 est associée à un renfort 41 (figures 1 et 2) pour former une zone de préhension 35 renforcée dédiée à l'extraction du ballon intra-gastrique 1. Le renfort 41 se présente avantageusement sous la forme d'une membrane flexible 42 qui s'étend le long de la zone de préhension 35 de telle manière qu'elle ne fait pas saillie à l'extérieur de l'enveloppe superficielle 4, 40 et ne forme donc aucune protubérance susceptible de porter atteinte à la géométrie régulière et atraumatique du ballon intra-gastrique 1.

De façon préférentielle, la forme du renfort 41 épouse sensiblement la forme de la collerette 5 et de l'enveloppe superficielle 4, 40 auxquelles il est associé. De façon encore plus préférentielle, le renfort 41 est superposé à la collerette 5 à l'intérieur du ballon intra-gastrique 1 et avantageusement collé ou soudé, selon toute sa surface, sur la surface interne 51 de la collerette 5. Le renfort 41 peut également se prolonger, tel que cela est illustré sur les figures 1 et 2, sous l'enveloppe superficielle 4, 40. Dans ce dernier cas, le renfort 41 est préférentiellement solidarisé à l'enveloppe superficielle 4, 40, par soudure ou collage avec la face interne 4I, 40I de ladite enveloppe superficielle 4, 40. Il est également envisageable, sans sortir du cadre de l'invention, de rapporter le renfort 41 sur la surface externe 5E de la collerette 5 ou même encore de noyer le renfort 41 dans l'épaisseur de la collerette 5. De la même façon, le renfort 41 pourra s'étendre sur la face externe 4E, 40E de l'enveloppe superficielle 4, 40 ou être noyé dans l'épaisseur de cette dernière.

Le renfort 41 comprend avantageusement au moins une pièce textile, comprenant par exemple un tulle de polyester, ou encore un matériau tissé

(ou non tissé) réalisé à partir de fibres de polyamide, et/ou de fibres d'aramide. On peut également envisager de mettre en oeuvre, en tant que pièce de renfort, une structure fibreuse de type *« nid d'abeilles »* bien connue en tant que telle.

Avantageusement, le moyen de connexion 3 comprend un organe de compression 50, tel qu'une bague, disposé de manière à entourer au moins l'une des cloisons auto-cicatrisantes 17 de manière à la comprimer latéralement. L'organe de compression 50 est avantageusement dimensionné pour exercer une compression radiale centripète sur ladite cloison auto-cicatrisante 17, suivant une direction sensiblement perpendiculaire à l'axe de perforation Z-Z', de manière à conférer à cette dernière son caractère étanche et auto-cicatrisant ou simplement contribuer à améliorer le caractère auto-cicatrisant intrinsèque de ladite cloison auto-cicatrisante 17.

De façon préférentielle, l'organe de compression 50 et l'élément de fixation 9 sont formés par un même élément, à savoir la bague 10.

Avantageusement, le moyen de connexion 3, et plus précisément le corps principal 30, se présente sous la forme d'un corps cylindrique allongé qui comporte un tronçon comprimé 33 comprenant au moins une cloison auto-cicatrisante 17, de préférence formée par la cloison auto-cicatrisante supérieure 17A, ainsi que la chambre 16 délimitée latéralement par les parois latérales 18. Le tronçon comprimé 33 est avantageusement comprimé latéralement et de manière centripète à l'aide de l'organe de compression 50, formé par la bague 10.

De façon préférentielle, la compression de la chambre 16 est comprise entre 10 % et 20 %.

La cloison auto-cicatrisante inférieure 17B est avantageusement rapportée sur le tronçon comprimé 33, et plus précisément sur les extrémités 18A des parois latérales 18, de manière à venir refermer de manière étanche la chambre 16. Sur la variante préférentielle illustrée sur la figure 4, la cloison auto-cicatrisante inférieure 17B n'est pas comprimée radialement par l'organe de compression 50 et se trouve dans un état relaxé.

De façon préférentielle, les parois latérales 18 de la chambre 16 sont venues de matière avec l'une des cloisons auto-cicatrisante, et de préférence avec la cloison auto-cicatrisante supérieure 17A, formant avec cette dernière un ensemble monobloc susceptible d'être obtenu par moulage.

De façon encore plus préférentielle, la collerette 5, la portion supérieure 30S et le tronçon comprimé 33 forment un ensemble monobloc obtenu par moulage, la cloison auto-cicatrisante inférieure 17B étant rapportée, par collage ou soudure, sur le tronçon comprimé 33 de manière à refermer la chambre 16.

Avantageusement, le moyen de connexion 3, et notamment le corps principal 30, comprend un organe d'étirement 36 apte à être manipulé pour assurer l'étirement axial, suivant l'axe longitudinal X-X', du moyen de connexion 3, et permettre le montage de l'organe de compression 50 autour d'au moins l'une des cloisons auto-cicatrisantes 17.

Ainsi, l'organe de compression 50 présente avantageusement un diamètre interne inférieur au diamètre externe au repos de la cloison auto-cicatrisante 17 à laquelle il est associé de manière à pouvoir comprimer radialement cette dernière.

L'étirement axial, suivant l'axe longitudinal X-X', de la cloison auto-cicatrisante 17 à laquelle est associé l'organe de compression 50 permet ainsi de faire diminuer le diamètre externe de la cloison auto-cicatrisante 17 jusqu'à ce que ce dernier atteigne le diamètre interne de l'organe de compression 50 et notamment de la bague 10. Il est alors possible d'emmancher la bague 10 sur la cloison auto-cicatrisante, et notamment sur la cloison auto-cicatrisante supérieure 17A, tel que cela est illustré sur la figure 4.

De façon préférentielle, l'organe d'étirement 36 est formé par une languette de traction 37 solidarisée, c'est-à-dire fixée sur ou venue de matière avec le moyen de connexion 3, et plus préférentiellement avec la cloison auto-cicatrisante inférieure 17B (figure 4). La languette de traction 37 forme ainsi avantageusement un prolongement, suivant l'axe longitudinal X-X', du corps principal 30.

L'invention concerne également un kit de mise en forme d'un ballon intra-gastrique 1 destiné à être implanté au sein de l'estomac d'un patient dans le cadre d'un traitement de l'obésité.

Selon l'invention, le kit comprend un ballon intra-gastrique 1 conforme à l'invention et un organe de connexion 11 du ballon intra-gastrique 1 à une source de fluide de gonflage, monté, dans sa configuration de stockage, de manière à transpercer la chambre 16 pour déboucher dans la poche 2. L'organe de connexion 11 comprend avantageusement une aiguille de gonflage 15 préférentiellement formée par une aiguille creuse apte à transpercer ladite chambre 16. Pendant toute la durée du stockage, l'aiguille de gonflage 15 est montée de manière à traverser de part en part la chambre 16 et les éventuelles cloisons auto-cicatrisantes 17 disposées en enfilade avec cette dernière.

La présente invention concerne également une nouvelle utilisation d'un fluide visqueux et/ou consistant en tant que joint d'étanchéité au sein d'un moyen de connexion 3, monté sur un ballon intra-gastrique 1 et conçu pour permettre le raccordement étanche du ballon intra-gastrique 1 à une source de fluide de gonflage.

De façon préférentielle, la viscosité et la consistance du fluide d'étanchéité sont telles que la pénétration à 25°C dudit fluide d'étanchéité par un mobile de 1 pouce et de 12 g, avec un temps de chute de 15 s, est inférieure à 20mm et de préférence comprise entre 10 mm et 18 mm.

De façon encore plus préférentielle, le fluide visqueux est formé par un gel, tel qu'un gel de silicone du type NuSil dont la référence est par exemple MED 12 6300.

La conception du ballon intra-gastrique 1 conforme à l'invention permet ainsi d'assurer, en toute sécurité, le remplissage du ballon intra-gastrique 1 à l'intérieur de l'estomac du patient, sans risque de fuite, et ce même après une période de stockage prolongée du ballon intra-gastrique 1 ou du kit comprenant, en association, le ballon intra-gastrique 1 et l'organe de connexion 11 monté sur le ballon.

Un autre avantage du ballon intra-gastrique 1 conforme à l'invention provient de sa facilité d'extraction, grâce à la présence de la cavité 31 dont la longueur est suffisante pour permettre le pincement du moyen de connexion 3 par une pince endoscopique.

### POSSIBILITE D'APPLICATION INDUSTRIELLE

L'invention trouve son application industrielle dans la fabrication et l'utilisation de ballons intra-gastriques de traitement de l'obésité.

## Revendications

1. Ballon intra-gastrique destiné à être implanté à l'intérieur de l'estomac d'un patient pour réduire le volume de l'estomac dans le cadre d'un traitement de l'obésité, ledit ballon intra-gastrique (1) comprenant :
- au moins une poche (2) apte à être remplie, au moins partiellement, avec un fluide de gonflage,
- un moyen de connexion (3), monté sur ladite poche (2) et apte à recevoir un organe de connexion (11) à une source de fluide de gonflage,
**caractérisé en ce que** le moyen de connexion (3) comporte une chambre (16) conçue pour être transpercée par ledit organe de connexion (11) en vue d'injecter le fluide de gonflage dans ladite poche (2), ladite chambre (16) renfermant un fluide d'étanchéité suffisamment visqueux et/ou consistant pour assurer l'étanchéité du moyen de connexion (3) une fois l'organe de connexion (11) retiré.

2. Ballon intra-gastrique selon la revendication 1 **caractérisé en ce que** le fluide d'étanchéité présente une viscosité et/ou une consistance suffisante pour piéger des particules de fluide de gonflage, par exemple des bulles ou des gouttes, susceptibles de se former dans la chambre (16) une fois l'organe de connexion (11) retiré.

3. Ballon intra-gastrique selon la revendication 1 ou 2 **caractérisé en ce que** la viscosité et la consistance du fluide d'étanchéité sont telles que la pénétration à 25°C dudit fluide d'étanchéité par un mobile de 1 pouce et de 12 g, avec un temps de chute de 15 s, est inférieure à 20 mm et de préférence comprise entre 10 mm et 18 mm.

4. Ballon intra-gastrique selon la revendication 1, 2 ou 3 **caractérisé en ce que** le fluide d'étanchéité est formé par un gel, par exemple un gel de silicone.

5. Ballon intra-gastrique selon l'une des revendications précédentes **caractérisé en ce que** le moyen de connexion (3) comporte au moins une cloison auto-cicatrisante (17), disposée en alignement axial avec la chambre (16) de manière à être transpercée par l'organe de connexion (11), ladite cloison auto-cicatrisante (17) étant suffisamment souple et élastique pour s'auto-obturer, une fois l'organe de connexion (11) retiré.

6. Ballon intra-gastrique selon la revendication 5 **caractérisé en ce que** le moyen de connexion (3) comporte au moins deux cloisons auto-cicatrisantes (17) disposées de part et d'autre de la chambre (16), en alignement axial avec cette dernière.

7. Ballon intra-gastrique selon la revendication 5 ou 6 **caractérisé en ce que** la chambre (16) comporte des parois de piquage (19), et **en ce que** les parois de piquage (19) sont formées par les cloisons auto-cicatrisantes (17).

8. Ballon intra-gastrique selon l'une quelconque des revendications 5 à 7 **caractérisé en ce que** le moyen de connexion (3) comprend un organe de compression (50), du genre bague, disposé de manière à entourer au moins l'une des cloisons auto-cicatrisantes (17) de manière à la comprimer latéralement.

9. Ballon intra-gastrique selon la revendication 8 **caractérisé en ce que** le moyen de connexion (3) comprend un organe d'étirement (36), apte à être manipulé pour assurer l'étirement axial du moyen de connexion (3), et permettre le montage de l'organe de compression (50) autour d'au moins l'une des cloisons auto-cicatrisantes (17).

10. Ballon intra-gastrique selon la revendication 9 **caractérisé en ce que** l'organe d'étirement (36) est formé par une languette de traction (37), fixée sur ou venue de matière avec le moyen de connexion (3).

11. Ballon intra-gastrique selon l'une des revendications précédentes **caractérisé en ce que** le moyen de connexion (3) comprend un corps principal (30), faisant saillie vers l'intérieur du ballon intra-gastrique (1), ledit corps principal (30) comprenant une portion supérieure (30S), évidée de manière à former une cavité (31), et une portion inférieure (301), au sein de laquelle est ménagée la chambre (16).

12. Ballon intra-gastrique selon la revendication 11 **caractérisé en ce que** la portion supérieure (30S) est évidée sur une distance suffisante pour que la paroi (32) du corps principal (30) délimitant la cavité (31) puisse se rabattre sur elle-même lors du pincement de la portion supérieure (30S) du corps principal (30) avec un outil d'extraction du ballon intra-gastrique (1), tel qu'une pince endoscopique.

13. Ballon intra-gastrique selon l'une des revendications précédentes **caractérisé en ce que** le moyen de connexion (3) comprend une collerette (5) s'étendant radialement à la surface du ballon intra-gastrique (1), de manière à former une zone de préhension (35) du ballon intra-gastrique (1) par l'outil d'extraction.

14. Ballon intra-gastrique selon la revendication 13 **caractérisé en ce que** la zone de préhension (35) est associée à un renfort (41), pour former une zone de préhension (35) renforcée dédiée à l'extraction du ballon intra-gastrique (1).

15. Kit de mise en forme d'un ballon intra-gastrique (1) destiné à être implanté au sein de l'estomac d'un patient dans le cadre d'un traitement de l'obésité comprenant :
- un ballon intra-gastrique selon l'une des revendications 1 à 14,
- un organe de connexion (11) du ballon intra-gastrique (1) à la source de fluide de gonflage, monté, dans sa configuration de stockage, de manière à transpercer ladite chambre (16) pour déboucher dans la poche (2).

16. Kit selon la revendication 15 **caractérisé en ce que** l'organe de connexion (11) comprend une aiguille de gonflage (15), apte à transpercer ladite chambre (16).

## Claims

1. Gastric balloon intended to be implanted inside the stomach of a patient in order to reduce the volume of the stomach as part of a treatment for obesity, the said gastric balloon (1) comprising:
- at least one pouch (2) that can be filled, at least partially, with an inflation fluid,
- a connection means (3), mounted on the said pouch (2) and which can accept a connection member (11) for connection to a source of inflation fluid,
**characterized in that** the connection means (3) comprises a chamber (16) designed to be transpierced by the said connection member (11) so that the inflation fluid can be injected into the said pouch (2), the said chamber (16) containing a sealing fluid that is sufficiently viscous and/or has sufficient consistency that it can seal the connection means (3) once the connection member (11) has been withdrawn.

2. Gastric balloon according to Claim 1, **characterized in that** the sealing fluid has sufficient viscosity and/or sufficient consistency that it can trap particles of inflation fluid, for example bubbles or droplets, liable to form in the chamber (16) once the connection member (11) has been withdrawn.

3. Gastric balloon according to Claim 1 or 2, **characterized in that** the viscosity and the consistency of the sealing fluid are such that the penetration of the said sealing fluid at 25°C by a moving body measuring 1 inch and weighing 12 g, with a time of fall of 15 s, is less than 20 mm and preferably comprised between 10 mm and 18 mm.

4. Gastric balloon according to Claim 1, 2 or 3, **characterized in that** the sealing fluid is formed of a gel, for example a silicone gel.

5. Gastric balloon according to one of the preceding claims, **characterized in that** the connection means (3) comprises at least one self-healing wall (17) positioned in axial alignment with the chamber (16) so that it is transpierced by the connection member (11), the said self-healing wall (17) being soft and elastic enough to self-seal once the connection member (11) has been withdrawn.

6. Gastric balloon according to Claim 5, **characterized in that** the connection means (3) comprises at least two self-healing walls (17) positioned on each side of the chamber (16) in axial alignment therewith.

7. Gastric balloon according to Claim 5 or 6, **characterized in that** the chamber (16) comprises injection walls (19), and **in that** the injection walls (19) are formed by the self-healing walls (17).

8. Gastric balloon according to any one of Claims 5 to 7, **characterized in that** the connection means (3) comprises a compression member (50), of the ring type, positioned so that it surrounds at least one of the self-healing walls (17) so as to compress it laterally.

9. Gastric balloon according to Claim 8, **characterized in that** the connection means (3) comprises a stretching member (36) able to be manipulated in order axially to stretch the connection means (3) and allow the compression member (50) to be fitted around at least one of the self-healing walls (17).

10. Gastric balloon according to Claim 9, **characterized in that** the stretching member (36) is formed of a pull tab (37), fixed to or formed as an integral part of the connection means (3).

11. Gastric balloon according to one of the preceding claims, **characterized in that** the connection means (3) comprises a main body (30) projecting towards the inside of the gastric balloon (1), the said main body (30) comprising an upper portion (30S) that is hollowed out in such a way as to form a cavity (31), and a lower portion (301) in which the chamber (16) is formed.

12. Gastric balloon according to Claim 11, **characterized in that** the upper portion (30S) is hollowed out over sufficient distance for the wall (32) of the main body (30) delimiting the cavity (31) to be able to be folded over on itself when the upper portion (30S) of the main body (30) is squeezed by a tool used to extract the gastric balloon (1), such as endoscopy forceps.

13. Gastric balloon according to one of the preceding claims, **characterized in that** the connection means (3) comprises a flange (5) extending radially at the surface of the gastric balloon (1), so as to form a region (35) via which the extraction tool can grasp the gastric balloon (1).

14. Gastric balloon according to Claim 13, **characterized in that** the grasping region (35) is associated with a reinforcement (41) to form a reinforced grasping region (35) dedicated to the extraction of the gastric balloon (1).

15. Kit for deploying a gastric balloon (1) intended to be implanted in the stomach of a patient as part of a treatment for obesity, comprising:
- a gastric balloon according to one of Claims 1 to 14,
- a connection member (11) for connecting the gastric balloon (1) to the source of inflation fluid and mounted, in its storage position, in such a way as to transpierce the said chamber (16) to emerge in the pouch (2).

16. Kit according to Claim 15, **characterized in that** the connection member (11) comprises an inflation needle (15) able to transpierce the said chamber (16).

## Patentansprüche

1. Intragastrischer Ballon für die Implantation in das Mageninnere eines Patienten zur Reduktion des Magenvolumens im Rahmen einer Behandlung gegen Fettleibigkeit, wobei der intragastrische Ballon (1) Folgendes umfasst:
- mindestens eine Tasche (2), die dafür ausgelegt ist, zumindest teilweise mit einem Aufblasfluid gefüllt zu werden,
- mindestens eine Verbindungseinrichtung (3), die an der Tasche (2) angebracht ist und dafür ausgelegt ist, ein Verbindungselement (11) mit einer Quelle für das Aufblasfluid aufzunehmen,
**dadurch gekennzeichnet, dass** die Verbindungseinrichtung (3) eine Kammer (16) besitzt, die dafür bestimmt ist, von dem Verbindungselement (11) durchstoßen zu werden, um das Aufblasfluid in die Tasche (2) zu injizieren, wobei die Kammer (16) ein Dichtungsfluid enthält, das ausreichend viskos und/oder dickflüssig ist, dass es die Dichtigkeit der Verbindungseinrichtung (3) gewährleistet, nachdem das Verbindungselement (11) zurückgezogen wurde.

2. Intragastrischer Ballon nach Anspruch 1, **dadurch gekennzeichnet, dass** das Dichtungsfluid eine ausreichende Viskosität und/oder Dickflüssigkeit aufweist, das es Teilchen des Aufblasfluids, zum Beispiel Blasen oder Tropfen, die sich in der Kammer (16) bilden können, abfängt, nachdem das Verbindungselement (11) zurückgezogen wurde.

3. Intragastrischer Ballon nach Anspruch 1 oder 2, **dadurch gekennzeichnet, dass** die Viskosität und die Konsistenz des Dichtungsfluids derart sind, dass die Penetration des Dichtungsfluids bei 25°C durch ein Gewicht von 1 Zoll und 12 g bei einer Fallzeit von 15 s kleiner als 20 mm und vorzugsweise zwischen 10 mm und 18 mm ist.

4. Intragastrischer Ballon nach Anspruch 1, 2 oder 3, **dadurch gekennzeichnet, dass** das Dichtungsfluid von einem Gel, beispielsweise einem Silikongel, gebildet wird.

5. Intragastrischer Ballon nach einem der vorhergehenden Ansprüche, **dadurch gekennzeichnet, dass** die Verbindungseinrichtung (3) mindestens eine selbstverschließende Trennwand (17) aufweist, die in axialer Ausrichtung mit der Kammer (16) derart angebracht ist, dass sie durch das Verbindungselement (11) durchstoßen wird, wobei die selbstverschließende Trennwand (17) ausreichend biegsam und elastisch ist, dass sie sich von selbst verschließt, nachdem das Verbindungselement (11) zurückgezogen wurde.

6. Intragastrischer Ballon nach Anspruch 5, **dadurch gekennzeichnet, dass** die Verbindungseinrichtung (3) mindestens zwei selbstverschließende Trennwände (17) aufweist, die auf beiden Seiten der Kammer (16) in axialer Ausrichtung mit dieser angebracht sind.

7. Intragastrischer Ballon nach Anspruch 5 oder 6, **dadurch gekennzeichnet, dass** die Kammer (16) Stechwände (19) aufweist, und **dadurch**, dass die Stechwände (19) durch selbstverschließende Trennwände (17) gebildet werden.

8. Intragastrischer Ballon nach einem der Ansprüche 5 bis 7, **dadurch gekennzeichnet, dass** die Verbindungseinrichtung (3) ein Kompressionselement (50) von der Art einer Hülse umfasst, das derart angebracht ist, dass es mindestens eine der selbstverschließenden Trennwände (17) derart umgibt, dass diese seitlich komprimiert wird.

9. Intragastrischer Ballon nach Anspruch 8, **dadurch gekennzeichnet, dass** die Verbindungseinrichtung (3) ein Streckelement (36) umfasst, das dafür ausgelegt ist, derart gehandhabt zu werden, dass die axiale Streckung der Verbindungseinrichtung (3) gewährleistet wird und die Anbringung des Kompressionselements (50) um mindestens eine der selbstverschließenden Trennwände (17) ermöglicht wird.

10. Intragastrischer Ballon nach Anspruch 9, **dadurch gekennzeichnet, dass** das Streckelement (36) durch eine Zuglasche (37) gebildet wird, die an der Verbindungseinrichtung (3) befestigt oder einstückig damit ausgeführt ist.

11. Intragastrischer Ballon nach einem der vorhergehenden Ansprüche, **dadurch gekennzeichnet, dass** die Verbindungseinrichtung (3) einen Hauptkörper (30) besitzt, der zum Inneren des intragastrischen Ballons (1) vorspringt, wobei der Hauptkörper (30) umfasst: einen oberen Abschnitt (30S), der ausgehöhlt ist, so dass ein Hohlraum (31) gebildet wird, und einen unteren Abschnitt (301), in dessen Innerem die Kammer (16) hergestellt ist.

12. Intragastrischer Ballon nach Anspruch 11, **dadurch gekennzeichnet, dass** der obere Abschnitt (30S) in einem ausreichenden Abstand ausgehöhlt ist, dass sich die Wand (32) des Hauptkörpers (30), welche den Hohlraum (31) abschließt, beim Durchstechen des oberen Abschnitts (30S) des Hauptkörpers (30) mit einem Werkzeug zur Extraktion des intragastrischen Ballons (1), wie einem Endoskop Zange, auf sich selbst umlegen kann.

13. Intragastrischer Ballon nach einem der vorhergehenden Ansprüche, **dadurch gekennzeichnet, dass** die Verbindungseinrichtung (3) einen Kragen (5) umfasst, der sich radial auf der Oberfläche des intragastrischen Ballons (1) erstreckt, so dass eine Greifzone (35) des intragastrischen Ballons (1) für das Extraktionswerkzeug gebildet wird.

14. Intragastrischer Ballon nach Anspruch 13, **dadurch gekennzeichnet, dass** die Greifzone (35) mit einer Versteifung (41) einhergeht, so dass eine verstärkte Greifzone (35) gebildet wird, die der Extraktion des intragastrischen Ballons (1) dient.

15. Kit zur Formgestaltung eines intragastrischen Ballons (1) für die Implantation in das Mageninnere eines Patienten im Rahmen einer Behandlung gegen Fettleibigkeit, umfassend:
- einen intragastrischen Ballon nach einem der Ansprüche 1 bis 14,
- ein Verbindungselement (11) des intragastrischen Ballons (1) mit der Quelle für Aufblasfluid, das in seiner Konfiguration während der Lagerung derart angebracht ist, dass es die Kammer (16) durchstößt, so dass es in die Tasche (2) mündet.

16. Kit nach Anspruch 15, **dadurch gekennzeichnet, dass** das Verbindungselement (11) eine Aufblasnadel (15) umfasst, die dafür ausgelegt ist, die Kammer (16) zu durchstoßen.
